# EUROPEAN PATENT SPECIFICATION

(11) **EP 0 999 824 B1**
(45) Date of publication and mention of the grant of the patent: **12.03.2003**
(21) Application number: 98925803.3
(22) Date of filing: 01.06.1998
(51) Int. Cl.: A61K 9/00, C22C 23/02

(54) **RELEASE DEVICES**
EINNAHMEVORRICHTUNGEN
DISPOSITIF DE LIBERATION

(30) Priority: 09.06.1997 GB 9711946
(43) Date of publication of application: 17.05.2000
(73) Proprietor: CASTEX PRODUCTS LIMITED, Stockport, Cheshire SK12 3HG (GB)
(72) Inventor: WHITEHEAD, Derek, James, Cheshire SK12 1NT (GB)
(74) Representative: Linn, Samuel Jonathan
(86) International application number: GB9801595
(87) International publication number: WO98056347

(56) References cited:
- EP-A- 0 164 927
- EP-A- 0 243 111
- EP-A- 0 482 947
- GB-A- 1 030 101
- US-A- 1 688 043
- PATENT ABSTRACTS OF JAPAN vol. 14, no. 544 (C-0784), 4 December 1990 & JP 02 232332 A (TECH RES & DEV INST OF JAPAN DEF AGENCY), 14 September 1990,

## Description

This invention relates to release devices in the form of pellets or boli, more particularly those comprising a magnesium alloy, for use in the treatment of ruminant animals for the prevention of hypomagnesaemic tetany. The invention further relates to methods of making the release devices and also to novel magnesium alloys for use in the making of such release devices.

United Kingdom Patent GB 1030101 discloses results of various trials in the treatment of ruminant animals for preventing hypomagnesaemia, in which pellets of magnesium-based alloys are used which contain from 5 to 20% by weight, preferably 8 to 20% by weight, of aluminium, together with various additions of copper, zinc, nickel and cobalt. This early work dating from the mid 1960s led to the development and commercial exploitation of boli based on the use of the alloy designated MAB1, which contains, by weight, approximately 12% aluminium, 2% copper, and the balance magnesium. Such boli are known for introduction into the rumeno-reticular sac of ruminant animals via the mouth, where they gradually dissolve by reaction with the rumen liquor, the released magnesium, in the form of Mg⁺⁺ ions, becoming available for assimilation by the animal.

A widely used commercial bolus of the above type for the treatment of hypomagnesaemia in cattle takes the form of a cylinder with rounded ends and which is approximately 7.5 cm long and 2.5 cm in diameter. The bolus is fabricated from MAB1 alloy, weighted with finely divided iron particles dispersed throughout the alloy matrix to give a bolus density of about 3 g/ml. This relatively high density prevents loss of the bolus by regurgitation and aids dissolution of the bolus by causing galvanic reaction between the iron and magnesium alloy in the presence on an electrolyte, which in this case is the rumen liquor.
Cattle with body weight in excess of 300 kg are normally given two boli prior to turn out on spring pasture when the risk of development of clinical tetany in response to hypomagnesaemia is highest. Each bolus typically contains 40 grams of magnesium, the total maximum magnesium intake thus being approximately 80 grams, together with approximately 11 grams aluminium and approximately 2 grams copper. The maximum release rate of magnesium from two boli of this type has been estimated at 1.3 grams per day.

A further type of magnesium-based bolus which has found commercial application for cattle is disclosed in AU-A-470538. The bolus disclosed there comprises two elongate, arcuate cross-sectioned castings of magnesium alloy measuring approximately 17 cm long and 3.5 cm wide, the two half castings being joined together by a flexible rubber hinge. The bolus is introduced as a cylinder into the rumen of the animal via the mouth, following which the rubber hinge opens lengthwise so as to present a wide cross-sectional area of the bolus which resists regurgitation by the animal. The commercial magnesium-based alloy designated AZ91 containing by weight approximately 9% aluminium, 1% zinc, and the balance magnesium, has been used for this device. One bolus is normally administered per animal and a study has indicated a maximum release rate of magnesium of 1.43 grams per day.

It has been estimated that a daily dietary magnesium requirement of about 20 grams magnesium is required to maintain magnesium blood serum values of 2 mg/100 ml in lactating cows, a level below which is associated with the occurrence of grass tetany. Generally the animals can receive around 13 grams per day of magnesium from their natural feed, which leaves an anticipated need of supplemental magnesium in excess of around 7 grams per day to ensure that a high level of protection against grass tetany is maintained.

Past experience indicates that in more borderline cases, i.e. cases in which the risk of hypomagnesaemia is less, the magnesium boli in current use are generally effective in controlling grass tetany. However, generally there is a distinct demand for a magnesium-based bolus which provides a higher magnesium release rate than is currently available, in order to cope with instances of more severe magnesium deficiency and to provide an enhanced level of protection against hypomagnesaemia.

It is known that other methods for increasing magnesium intake by ruminant animals are available, for example by top-dressing the pasture upon which they feed with minerals such as magnesite or dolomite, or the addition of magnesium compounds to feed blocks, loose feed concentrates or drinking water. Any novel bolus product must therefore be price-competitive, even taking into account the convenience of a one dose regime, which may be effective for several weeks. It is not feasible to achieve higher magnesium release rates by simply increasing the number of boli administered to a ruminant animal, because of cost and practical difficulties. It is furthermore undesirable to increase the quantity of non-biodegradable bolus components which may be left in the rumen after the boli have disintegrated. Therefore, any new bolus design should preferably fulfil many if not most or all of the following characteristics:
(1) Possess a substantially higher magnesium release rate than is available from currently used boli.
(2) Give a practically useful bolus life.
(3) Be fully biodegradable and leave substantially no permanent residue in the rumen of the animal.
(4) Be economical to produce.
(5) Be substantially free from toxic alloying elements at harmful or undesirable levels.
(6) Be sufficiently dense to resist regurgitation by the animal to which the bolus is administered.

The known MAB1 alloy is used in other commercially available boli whose function is the controlled release of anthelimintics to ruminant animals. The rate of galvanic corrosion of the alloy in the rumen effectively provides a timing mechanism which governs the release of a biologically active material contained within the bolus. Such is disclosed for example in EP-A-0164927, which document teaches how release of the biologically active agent can be achieved as a series of discrete pulsed releases.

In contrast, EP-A-0243111 discloses a tubular bolus for achieving sustained continuous release. This reference also teaches the use of graphite-bearing tablets or cores contained within a magnesium alloy tube for functioning as reactive cathodes when introduced into the rumen of an animal. However, the amount of magnesium released by the devices disclosed in this reference has been found to be too small to be of significant benefit in combatting hypomagnesaemia.

Surprisingly, following studies on the opposing effects of aluminium and copper in controlling the corrosion of magnesium-aluminium-copper alloys in dilute salt solutions, we have found that boli containing high aluminium contents such as have traditionally been used for fabricating magnesium-based bolus alloys such as MAB1 and AZ91, tend to have a retarding effect on their corrosion and reduce the accelerating influence of copper on the corrosion rate. As a result, we have devised a novel magnesium alloy-based bolus for veterinary use in the treatment of hypomagnesaemia in ruminant animals, which is characterised by a higher magnesium release rate in the rumen and additionally is capable of providing a higher total dose of available magnesium than has hitherto been the case with known boli employing known magnesium-based alloys. Further studies into the effects of differing levels of aluminium and copper on relevant alloy properties such as castability and toxicity have also led us to the devising of novel magnesium-based alloys having an optimized range of chemical, biological and physical properties, for use in making such boli.

Accordingly, in a first aspect the present invention provides a release device in the form of a bolus for administration to a ruminant animal by deposition in its rumeno-reticular sac, the bolus comprising a metal alloy, which metal alloy comprises:
(a) a major proportion of magnesium;
(b) aluminium in an amount of less than 8% by weight of the alloy; and
(c) copper to enhance the rate of galvanic corrosion of the alloy, present in an amount of at least about 0.5% by weight of the alloy.

Preferred boli according to the first aspect of the invention further comprise an amount of a high specific gravity weight material in order that the resulting bolus has an overall density of at least about 2 g/cm³, more preferably at least about 2.5 or 3 g/cm³. Suitable high specific gravity materials include for example metal in the form of powder, granules or shot. Suitable metals for this purpose include for instance iron, copper, nickel, zinc, tungsten and alloys of any of the aforesaid metals, or possibly other metals or alloys thereof with high specific gravities provided they do not exhibit harmful or undesirable toxicities. In one simple form of bolus according to the first aspect of the invention, the bolus comprises an elongate moulded or extruded, preferably cylindrical body of a composition comprising a metal alloy as defined with reference to the first aspect of the invention having dispersed therein, preferably evenly there throughout, particles or bodies of the high specific gravity weight material, e.g. iron shot, such that the overall bolus composition has a density at least about 2.5 or 3 g/cm³.

In a more elaborate form of bolus according to the first aspect of the invention, and in accordance with a second generally defined aspect of the present invention, there is provided a release device in the form of a generally elongate bolus for administration to a ruminant animal by deposition in its rumeno-reticular sac, the bolus comprising a magnesium alloy component in the form of a tubular sheath surrounding a degradable core component, the degradable core component including an electrically conductive material such that the degradable core component as a whole is electrically conductive and is galvanically coupled to the magnesium alloy sheath component, wherein the magnesium alloy component comprises a metal alloy as defined above with reference to the first aspect of the invention.

Boli formulated and constructed according to the first and (especially) second aspects of the present invention can give rise to relatively high magnesium release rates as well as providing an overall higher proportion of available magnesium for assimilation by the animal. This is achieved, according to the general underlying essence of the invention, by using significantly lower aluminium contents in the copper (or other corrosion-promoting metal)-containing magnesium alloy than has hitherto been used in the art. By using a dense conductive cathodic core in the bolus according to the second aspect of the invention, it is also possible to avoid or substantially prevent the leaving of permanent residues from the bolus in the rumen once it has degraded.

Preferred boli in accordance with the second aspect of the invention are in the form of elongate tubular bodies, preferably circular in cross section, with the magnesium alloy component in the form of a cylindrical sheath surrounding the central degradable core. The sheath may or may not be capped at one end in order to extend the life of the bolus.

In the bolus of the invention into which the magnesium-based alloy is incorporated, the component which enhances the rate of galvanic corrosion of the alloy is copper but may be additionally any metal or combination of metals which promotes such corrosion when the bolus is in use as compared to the case where that metal(s) is absent. Most preferred is copper alone. Other metals which perform similarly in promoting galvanic corrosion may however be used additionally, with the general proviso that they are only used, and are used in amounts, such that they do not impart undesirable or harmful properties to the resulting alloy, e.g. toxicity. Suitable metals for this purpose include for instance nickel and calcium.

The magnesium alloys which may form the magnesium alloy components of the preferred boli according to the invention, comprise, in addition to magnesium (in the form of magnesium metal), less than 8% by weight aluminium and copper (and optionally other galvanic corrosion-enhancing metal) in an amount of at least about 0.5% by weight of the alloy composition. The amount of copper may be more than 0.5 % by weight. More preferably, aluminium is present in the alloy in the range of from about 0.5 to about 7.9 or 7.95% by weight, even more preferably from about 0.5 to about 7.75 or 7.8% by weight, most preferably in the range of from about 1 to about 7.5% by weight of the composition Particularly preferred amounts of copper (and optionally other galvanic corrosion-enhancing metal) in the composition are those in the range of from about 0.5 to about 5% by weight, more preferably from about 0.5 to about 4% by weight.

In practical examples of the magnesium-rich alloy of the invention, the amounts of aluminium and copper (or other corrosion-promoting metal) actually incorporated will generally be chosen so as to optimize a balance of contra-dependent properties, in particular corrodability, low toxicity and castability or ease of extrusion. In this respect the relative amounts of aluminium and copper ( and optionally other corrosion-promoting metal) may be important. For example, lower levels of aluminium (e.g. down to around 2% by weight) help to promote corrodability in conjunction with given moderate levels (e.g. 2 or 3% by weight) of copper, but such a lowering of amounts of both elements can lead to problems with castability of the alloy and such alloys may be better extruded as a tube rather than shape cast. Copper can lead to toxicity problems when present at higher levels (e.g. 5% by weight), so such levels are undesirable when demanded by correspondingly higher levels of aluminium (e.g. around 8% by weight).

According to the present invention the combined aluminium and galvanic corrosion-enhancing metal (e.g. copper) contents in the alloy of the magnesium alloy component are preferably such that the resulting bolus gives a magnesium release rate, in accordance with the in vitro test procedure described hereinbelow, of at least about 2 grams/day, more preferably at least about 3, grams/day, most preferably from about 3 to about 11 or 12 grams/day. By suitable adjustment of the formulation of the magnesium alloy component, especially by suitable lowering of the amount of aluminium present, particularly good magnesium release rates may be achieved. Particular desired magnesium release rate values for any particular technical application may thus be achieved by appropriate selection and control of the formulation, especially the aluminium content, of the magnesium alloy component of the bolus.

In practical embodiments of the preferred bolus of the invention the magnesium alloy sheath can be made by any suitable process known in the art, especially by die casting or alternatively by extrusion. Exemplary processes for this purpose are disclosed for example in EP-A-0164927 and EP-A-0243111 mentioned hereinabove. In the case of a die cast tubular sheath, the bore is preferably tapered to allow extraction of the core once the tube is formed. The taper can thereafter be removed for example by drilling. Die cast tubular sheaths will normally have any exterior flash removed by a grinding or a barrelling operation which also serves to clean the exterior surface and remove any adherent oxide film.

In the preferred bolus of the invention the degradable core component includes at least one electrically conductive material such that the degradable core component as a whole is electrically conductive and is galvanically coupled to the magnesium alloy component, whereby the degradable core component forms a conductive cathodic core which degrades when introduced into the rumen, in the manner already known from the prior art.

The electrically conductive material is preferably selected from carbon, graphite, plumbago or mixtures thereof, any of which materials may suitably be in the form of granules, powder or short fibres.

The core material preferably further includes the amount of the high specific gravity weight material in order that the resulting bolus has an overall density of at least about 2 g/cm³, more preferably at least about 2.5 g/cm³. Suitable high specific gravity weight materials include for example metal powder, granules or shot. Suitable metals for this purpose include for instance iron, copper, nickel, tungsten, zinc or alloys of any of the aforesaid metals.

The degradable core material preferably further includes one or more additional ballast materials, especially in order to enhance the solubility of the core component in the rumen of the animal and/or to assist in binding the components of the core composition together. Powdered sucrose is particularly useful for this purpose. Other suitable ballast materials include stearates, urea, starch, lactose, and polymers such as polyvinylpyrrolidone.

The degradable core component of the bolus is preferably formed as a dense core by first preparing a mixture of the core component materials and then subjecting the mixture to high pressure. In one form of the core, a series of tablets are produced from the mixture of core materials and the tablets are thereafter packed into the tubular sheath in a manner similar to that already known in the art, e.g. from EP-A-0243111. Each tablet may be preformed on a rotary tablet press at a pressure for example of 20 kg/mm². Alternatively, the core component mixture may be forced directly into the preformed tubular sheath in successive stages, the mixture being compressed after each stage using a suitable press.

Practical examples of boli in accordance with the present invention may typically be made as unitary elongate tubular bodies, or alternatively may be made in two separate halves which may be administered to the animal separately using a suitable balling gun, or alternatively they may be joined together for single administration using a thin external degradable tube, e.g. of cardboard, which rapidly disintegrates in the animal. A typical size of bolus is a cylindrical tube measuring approximately 16 cm long with an external diameter of approximately 3.5 cm. Of course, however, any suitable size of bolus may be selected, for instance depending upon the age and size of animal in question.

In addition to the increased magnesium delivery rate using a bolus according to the present invention, a further advantage of the lower aluminium content magnesium alloy which forms the tubular sheath is that overall increased doses of magnesium for assimilation by the animal may be achieved for a bolus of a given total magnesium content. This results from the effect of aluminium in limiting the availability of the full magnesium content of the alloy to the animal. Aluminium is present in the microstructure of magnesium-aluminium alloys in two forms, namely in solid solution in magnesium, and secondly as the intermetallic compound Mg₁₇Al₁₂. The latter is insoluble in the rumen liquor and is eliminated in the animal faeces when the bolus corrodes. The compound is present in magnesium alloy as a degenerate eutectic and first appears in the microstructure at an aluminium content of around 2% by weight, increasing in volume with increasing aluminium content. Without being bound by any theory, it may be presumed therefore that in the case of for example a 12% by weight aluminium alloy, some 10% by weight is present as the intermetallic compound, which thus ties up about 6% (calculated using the relative atomic weights of Mg and Al) of the magnesium content of the alloy in an insoluble form which is unavailable to the animal.

It is within the scope of the present invention for the magnesium alloy component to include one or more additional elements, for example selenium in an amount of up to about 0.5% by weight and/or zinc in an amount of up to about 5% by weight.

Boli according to the present invention may provide an additional useful function, as well as for the treatment of hypomagnesaemia, for the delivery of additional therapeutic or pharmaceutical agents to the animal, by means of their incorporation into the magnesium alloy component or (more preferably, in the context of the preferred boli) the material of the degradable core component of the preferred form of boli. Useful therapeutic or pharmaceutical agents may include for example any of the following: trace elements such as additional copper (e.g. in the form of copper oxide), selenium (e.g. in metallic form or as selenium oxide or in the form of soluble salts such as selenites), iodine (e.g. in the form of alkali metal iodide or iodate), manganese (e.g. in metallic form or in the form of one or more salts), zinc (e.g. in metallic form or in the form of one or more salts) or cobalt (e.g. in the form of cobalt oxide or a soluble salt); vitamins; antibiotics; growth promoters; biologically active agents such as anthelmintics, antiparasitics and endectocides, including for example benzimidazoles and avermectins. Further particular therapeutic or pharmaceutical agents suitable for inclusion in the boli of the present invention include for example any of those disclosed in our earlier European Patent application EP-A-0164927 mentioned hereinabove, the content of which is incorporated herein by reference.

In a third aspect of the present invention, there is provided a method of making a bolus according to the second aspect of the invention, comprising any of the variously defined and described methods hereinabove and hereinbelow.

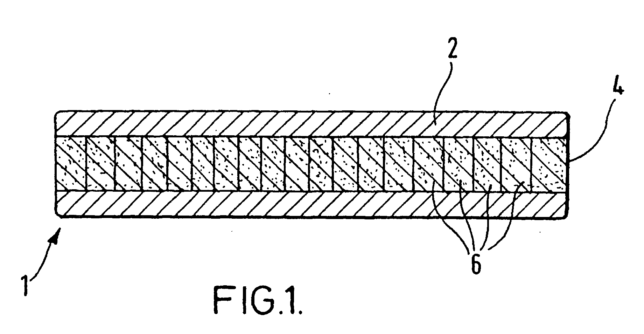

Preferred embodiments and features of the invention in its various aspects will now be described in detail further below, including with reference to the accompanying drawing in which the sole Figure 1 is a part-sectional view of a preferred construction of bolus in accordance with the invention.

The development of boli where the release mechanism is controlled by the corrosion of magnesium is considerably aided by the use of an in vitro test procedure which has been shown to closely mimic the dissolution of such boli in cattle. This minimises the need to use test animals which must either be slaughtered or have fistulated rumen in order to recover the test samples and evaluate the rate of dissolution in in vivo tests. The in vitro test procedure employed herein is as follows:

The test bolus is immersed in 0.1% by weight aqueous sodium chloride solution saturated with magnesium hydroxide and maintained at 35°C. The test bolus is maintained in the corrodant solution for a predetermined length of time and the bolus is then removed, rinsed in clean water, weighed and then the average magnesium release rate is calculated.

The invention is illustrated by way of the following examples.

### EXAMPLES

### Example 1

Figure 1 of the accompanying drawings illustrates a preferred constructional form of bolus according to the invention. The bolus, indicated generally as 1, comprises a tubular sheath 2 of magnesium-rich alloy having the follow approximate composition: aluminium 4% by weight, copper 3% by weight, balance magnesium. The tube 2 is preformed by die casting a molten mixture of the alloy components. The tubular sheath 2 is initially cast with a slight taper which is subsequently removed by drilling. Any exterior flash from the cast is removed by grinding. Within the tubular magnesium-rich alloy sheath 2 is a dense conductive core filling 4, in the form of a series of tablets 6 preformed from the mixture of core components on a rotary tablet press at a pressure of 20 kg/mm². The composition of the core mixture is as follows:

| | % by weight |
|---|---|
| Iron powder (100 mesh) | 81.5 |
| Graphite powder (100 mesh) | 16 |
| Sucrose powder (300 mesh) | 2.5 |

The pressed mixture forming the tablets 6 of the core component 4 has a density of 4.7 g/ml. The resulting bolus measures approximately 16 cm long with an external diameter of approximately 3.5 cm. It contains approximately 190 g of magnesium alloy and has an overall density of approximately 2.7 g/ml.

### Example 2

Another bolus in accordance with the present invention was made using the same magnesium alloy and manufacturing process as in Example 1, except here the degradable core component mixture had the following composition:

| | % by weight |
|---|---|
| Iron powder (100 mesh) | 79.4 |
| Graphite powder (100 mesh) | 15.8 |
| Sucrose powder (100 mesh) | 2.4 |
| Copper oxide | 2.165 |
| Cobalt oxide | 0.035 |
| Selenium (metal) | 0.026 |
| Potassium iodide | 0.174 |

The pressed tablets of the above core component mixture had a density of 5.0 g/ml.

### Example 3 (In vitro corrosion tests)

Sample boluses made according to Example 1 above, but including various different magnesium alloys as the tubular sheath material, were subjected to the in vitro corrosion test procedure as described above. The sample boluses had dimensions 4.5 cm long, 4.0 cm in diameter. The average magnesium release rate of each bolus was calculated and the results are shown in Table 1 below.

**Table 1**

| In vitro test results on various alloy compositions | | | |
|---|---|---|---|
| Alloy No. | Composition⁽¹⁾ | | Average Release Rate |
| | Al% | Cu% | gm/day |
| 6 | 1.1 | 1.0 | 6.4 |
| 16 | 1.1 | 2.0 | 9.4 |
| 17 | 1.0 | 3.0 | 11.1 |
| 7 | 2.0 | 0.8 | 5.2 |
| 18 | 2.0 | 2.1 | 11.1 |
| 19 | 2.0 | 3.0 | 11.7 |
| 8 | 3.0 | 0.9 | 5.4 |
| 20 | 3.0 | 2.0 | 8.4 |
| 21 | 3.1 | 3.0 | 9.5 |
| 1 | 4.4 | 0.6 | 4.0 |
| 2 | 4.5 | 0.9 | 3.6 |
| 3 | 4.3 | 1.4 | 3.8 |
| 4 | 4.3 | 2.0 | 6.0 |
| 5 | 4.4 | 2.5 | 7.4 |
| 13 | 4.1 | 3.0 | 9.1 |
| AZ63⁽²⁾ | 6.0 | 0 | 0 |
| 22 | 5.7 | 2.1 | 3.8 |
| 23 | 5.7 | 3.0 | 5.8 |
| 24 | 5.8 | 3.9 | 7.5 |
| 25 | 7.8 | 2.1 | 4.2 |
| 26 | 7.5 | 3.1 | 4.7 |
| 27 | 7.6 | 4.2 | 6.0 |
| AZ91 | 9.2 | 0⁽³⁾ | 0 |
| 11 | 8.8 | 1.5 | 3.2 |
| 10 | 8.9 | 2.0 | 4.9 |
| 30 | 9.0 | 3.0 | 4.7 |
| 28 | 8.8 | 4.1 | 5.9 |
| MAB1 | 11.7 | 2.1 | 4.3 |
| 29 | 12.2 | 3.2 | 4.0 |

| | | | |
|---|---|---|---|
| ⁽¹⁾ By weight %, balance magnesium | | | |
| ⁽²⁾ Known magnesium-based alloy, although not hitherto used in bolus manufacture; contains 3% zinc | | | |
| ⁽³⁾ Contains 1% zinc | | | |

It can be readily seen from the above results that aluminium and copper have opposing effects on controlling the corrosion of magnesium-aluminium-copper alloys in dilute salt solution. As the results show, high aluminium contents such as have traditionally been used for fabricating magnesium bolus alloys of the prior art, e.g. MAB1, AZ91, retard corrosion and reduce the accelerating influence of copper on the corrosion rate.

### Example 4 (In vivo corrosion tests)

Various sample boluses according to Example 1, but utilising different magnesium alloys for the tubular sheath material were subjected to an in vivo test in which each test bolus was placed in the reticulum of dairy cows and retrieved in a conventional manner at varying time intervals and weighed to evaluate the magnesium alloy loss, from which the average magnesium release rate was calculated. The results are shown in Table 2 below.

**Table 2**

| In vivo test results on selected alloy compositions | | | |
|---|---|---|---|
| Alloy | Composition⁽⁴⁾ | | Average release rate |
| | Al% | Cu% | gm/day |
| MAB1 | 11.7 | 2.1 | 1.0⁽⁵⁾ |
| MAB1 | 11.7 | 2.1 | 1.1⁽⁵⁾ |
| MAB1 | 11.7 | 2.1 | 1.0⁽⁵⁾ |
| 11 | 8.8 | 1.5 | 1.2⁽⁵⁾ |
| 11 | 8.8 | 1.5 | 1.3⁽⁵⁾ |
| 11 | 8.8 | 1.5 | 1.3⁽⁵⁾ |
| 11 | 8.8 | 1.5 | 1.5⁽⁵⁾ |
| 13 | 3.7 | 2.6 | 5⁽⁶⁾ |
| 31 | 5.7 | 1.7 | 3.06⁽⁷⁾ |
| 32 | 4.9 | 1.7 | 3.86⁽⁷⁾ |

| | | | |
|---|---|---|---|
| Notes ⁽⁴⁾ Weight %. Balance magnesium | | | |
| (5) Half boli. Test duration 37 days | | | |
| (6) Half boli. Four tests. All boli finished reacting between 26 and 37 days exposure. Release rate of each bolus 4-6 gm/day | | | |
| (7) Half boli. Test duration 21 days | | | |

The above in vivo results demonstrate the obtaining of relatively high magnesium release rates by use of substantially lower aluminium contents in the magnesium alloy component in comparison with the prior art. For instance, a bolus containing 3.7 % by weight aluminium and 2.6 % by weight copper is capable of delivering about 10 grams per day of magnesium, which is adequate for cases of severe magnesium deficiency.

These data furthermore illustrate the ability to formulate magnesium alloys for use in boluses according to the invention which are specially designed to have intermediate corrosion rates, for example where this is desirable according to the particular intended application, e.g. for extend bolus life.

## Claims

1. A release device in the form of a bolus for administration to a ruminant animal by deposition in its rumeno-reticular sac, the bolus comprising a metal alloy, which metal alloy comprises:
(a) a major proportion of magnesium;
(b) aluminium in an amount of less than 8% by weight of the alloy; and
(c) copper to enhance the rate of galvanic corrosion of the alloy, present in an amount of at least 0.5% by weight of the alloy.

2. A release device according to claim 1, wherein the aluminium is present in the alloy in an amount in the range of from 1 to 7.5% by weight of the alloy.

3. A release device according to claim 1 or claim 2, wherein the copper is present in an amount of up to 5% by weight of the alloy.

4. A release device according to any preceding claim, wherein the metal alloy consists, by weight of the alloy, of
(a) a major proportion of magnesium;
(b) aluminium in an amount of less than 8% by weight;
(c) copper in an amount of at least 0.5% by weight;
(d) selenium in an amount of 0 to 0.5% by weight; and
(e) zinc is an amount of 0 to 5% by weight.

5. A release device according to claim 4, wherein the metal alloy consists, by weight of the alloy, of
(a) a major proportion of magnesium;
(b) aluminium in an amount of from 1 to 7.5% by weight; and
(c) copper in an amount of from 0.5 to 5% by weight.

6. A release device according to claim 4 or claim 5, wherein the copper is present in an amount of from 0.5 to 4% by weight of the alloy.

7. A release device according to any one of claims 4 to 6, wherein the amount of aluminium is 2% by weight, and the amount of copper is 2 - 3% by weight, of the alloy.

8. A release device according to any preceding claim, further comprises an amount of a high specific gravity weight material such that the resulting bolus has an overall density of at least 2 g/cm³.

9. A release device according to claim 8, wherein the high specific gravity weight material in the bolus is a metal in the form of powder, granules or shot and selected from iron, copper, nickel, zinc, tungsten or alloys comprising any of the aforesaid metals.

10. A release device according to claim 8 or claim 9, in the form of a bolus comprising an elongate moulded or extruded body of a composition comprising the said metal alloy and having dispersed therein the high specific gravity weight material such that the overall bolus composition has a density of at least 2.5 g/cm³.

11. A release device according to claim 8, wherein the bolus comprises a tubular sheath component of the magnesium alloy, which tubular sheath component surrounds a degradable core component, the degradable core component including an electrically conductive material such that the degradable core component as a whole is electrically conductive and is galvanically coupled to the tubular sheath component.

12. A release device according to claim 11, wherein the degradable core component is in the form of an elongate body of circular cross section.

13. A release device according to any one of claims 8 to 12, wherein the combined aluminium and copper contents in the said alloy are such that the resulting bolus gives a magnesium release rate, as measured by the in vitro test procedure described herein, of at least 2 grams/day.

14. A release device according to claim 11 or claim 12, wherein the electrically conductive material in the degradable core component is selected from carbon, graphite, plumbago or mixtures thereof.

15. A release device according to claim 11 or claim 12, wherein the degradable core component material further includes an amount of a high specific gravity weight material such that the resulting bolus has an overall density of at least 2 g/cm³.

16. A release device according to any one of claims 11, 12, 14 and 15, wherein the degradable core component material further includes one or more additional ballast materials for enhancing the solubility of the core component in the rumen of the animal and/or to assist in binding the components of the core composition together, the said one or more ballast materials being selected from powdered sucrose, stearates, urea, starch, lactose and polymers.

17. A release device according to any one of claims 8 to 16, wherein the bolus further includes one or more additional therapeutic or pharmaceutical agents selected from any of the following: trace elements; vitamins; antibiotics; growth promoters; and biologically active agents such as anthelmintics, antiparasitics and endectocides.

18. Use, in the manufacture of a release device in the form of a bolus for administration to a ruminant animal by deposition in its rumeno-reticular sac, of an alloy comprising a magnesium/aluminium alloy containing a major proportion of magnesium, wherein
(i) the amount of aluminium in the alloy is less than 8% by weight; and
(ii) the amount of copper in the alloy is at least 0.5% by weight, for increasing the magnesium release rate.

## Patentansprüche

1. Freisetzungsvorrichtung in Form eines Bolus zur Verabreichung an einen Wiederkäuer durch Ablagerung in seinem Pansen-Netzmagen, wobei der Bolus eine Metalllegierung umfasst, welche Metalllegierung Folgendes umfasst:
(a) einen Hauptanteil aus Magnesium;
(b) Aluminium in einer Menge von weniger als 8 Gew.-% der Legierung; und
(c) Kupfer, um die Rate der galvanischen Korrosion der Legierung zu erhöhen, in einer Menge von zumindest 0,5 Gew.-% der Legierung.

2. Freisetzungsvorrichtung nach Anspruch 1, worin das Aluminium in der Legierung in einer Menge im Bereich von 1 bis 7,5 Gew.-% der Legierung enthalten ist.

3. Freisetzungsvorrichtung nach Anspruch 1 oder 2, worin das Kupfer in einer Menge von bis zu 5 Gew.-% der Legierung enthalten ist.

4. Freisetzungsvorrichtung nach einem der vorangegangenen Ansprüche, worin die Metalllegierung, bezogen auf das Gewicht der Legierung, aus Folgendem besteht:
(a) einem Hauptanteil aus Magnesium;
(b) Aluminium in einer Menge von weniger als 8 Gew.-%;
(c) Kupfer in einer Menge von zumindest 0,5 Gew.-%;
(d) Selen in einer Menge von 0 bis 0,5 Gew.-%; und
(e) Zink in einer Menge von 0 bis 5 Gew.-%.

5. Freisetzungsvorrichtung nach Anspruch 4, worin die Metalllegierung, bezogen auf das Gewicht der Legierung, aus Folgendem besteht:
(a) einem Hauptanteil aus Magnesium;
(b) Aluminium in einer Menge von 1 bis 7,5 Gew.-%; und
(c) Kupfer in einer Menge von 0,5 bis 5 Gew.-%.

6. Freisetzungsvorrichtung nach Anspruch 4 oder 5, worin das Kupfer in einer Menge von 0,5 bis 4 Gew.-% der Legierung enthalten ist.

7. Freisetzungsvorrichtung nach einem der Ansprüche 4 bis 6, worin die Aluminiummenge 2 Gew.-% und die Kupfermenge 2 bis 3 Gew.-% der Legierung ausmachen.

8. Freisetzungsvorrichtung nach einem der vorangegangenen Ansprüche, die weiters eine solche Menge eines Materials mit hohem spezifischem Gewicht umfasst, dass der resultierende Bolus eine Gesamtdichte von zumindest 2 g/cm³ aufweist.

9. Freisetzungsvorrichtung nach Anspruch 8, worin das Material mit hohem spezifischem Gewicht im Bolus ein Metall in Form eines Pulvers, Granulate oder Schrots ist und aus Eisen, Kupfer, Nickel, Zink, Wolfram oder eines dieser Metalle umfassenden Legierungen besteht.

10. Freisetzungsvorrichtung nach Anspruch 8 oder 9 in Form eines Bolus, der einen länglichen, geformten oder extrudierten Körper mit einer Zusammensetzung umfasst, die die Metalllegierung umfasst und in der das Material mit hohem spezifischem Gewicht so dispergiert ist, dass die Gesamt-Boluszusammensetzung eine Dichte von zumindest 2,5 g/cm³ aufweist.

11. Freisetzungsvorrichtung nach Anspruch 8, worin der Bolus eine röhrenförmige Hüllenkomponente aus der Magnesiumlegierung umfasst, wobei die röhrenförmige Hüllenkomponente eine abbaubare Kernkomponente umgibt, wobei die abbaubare Kernkomponente ein elektrisch leitendes Material umfasst, so dass die abbaubare Kernkomponente insgesamt elektrisch leitend und mit der röhrenförmigen Hüllenkomponente galvanisch verbunden ist.

12. Freisetzungsvorrichtung nach Anspruch 11, worin die abbaubare Kernkomponente in Form eines länglichen Körpers mit kreisförmigem Querschnitt vorliegt.

13. Freisetzungsvorrichtung nach einem der Ansprüche 8 bis 12, worin der kombinierte Gehalt an Aluminium and Kupfer in der Legierung ein solcher ist, dass der resultierende Bolus bei Messung nach dem hierin beschriebenen In-vitro-Testverfahren eine Magnesiumfreisetzungsrate von zumindest 2 g/Tag ergibt.

14. Freisetzungsvorrichtung nach Anspruch 11 oder 12, worin das elektrisch leitende Material in der abbaubaren Kernkomponente aus Kohlenstoff, Graphit, Reißblei oder Gemischen davon ausgewählt ist.

15. Freisetzungsvorrichtung nach Anspruch 11 oder 12, worin das Material der abbaubaren Kernkomponente weiters eine solche Menge eines Materials mit hohem spezifischem Gewicht umfasst, ddass der resultierende Bolus eine Gesamtdichte von zumindest 2 g/cm³ aufweist.

16. Freisetzungsvorrichtung nach einem der Ansprüche 11, 12, 14 und 15, worin das Material der abbaubaren Kernkomponente weiters ein oder mehrere zusätzliche Ballastmaterialien zur Erhöhung der Löslichkeit der Kernkomponente im Pansen des Tieres und/oder zum Unterstützen der Bindung der Komponenten der Kernzusammensetzung aneinander umfasst, wobei das eine oder die mehreren Ballastmaterial(ien) aus pulverförmiger Saccharose, Stearaten, Harnstoff, Stärke, Lactose und Polymeren ausgewählt ist bzw. sind.

17. Freisetzungsvorrichtung nach einem der Ansprüche 8 bis 16, worin der Bolus weiters ein oder mehrere zusätzliche therapeutische oder pharmazeutische Mittel umfasst, die aus beliebigen der Folgenden ausgewählt sind: Spurenelementen; Vitaminen; Antibiotika; wachstumsfördernden Mitteln; and biologisch aktiven Mitteln, wie z.B. Anthelmintika, Antiparasitika und Endektoziden.

18. Verwendung einer Legierung, die eine Magnesium/Aluminium-Legierung umfasst, die einen Hauptanteil an Magnesium enthält, worin
(i) die Aluminiummenge in der Legierung unter 8 Gew.-% liegt; und
(ii) die Kupfermenge in der Legierung zumindest 0,5 Gew.-% beträgt, um die Magnesiumfreisetzungsrate zu erhöhen,
zur Herstellung einer Freisetzungsvorrichtung in Form eines Bolus zur Verabreichung an einen Wiederkäuer lurch Ablagerung in seinem Pansen-Netzmagen.

## Revendications

1. Dispositif de libération sous la forme d'un bol pour l'administration à un animal ruminant par un dépôt dans son sac rumeno-réticulaire, le bol comprenant un alliage métallique, cet alliage métallique comprenant:
(a) une proportion principale de magnésium;
(b) l'aluminium en une quantité inférieure à 8% en poids de l'alliage; et
(c) du cuivre pour renforcer le taux de corrosion galvanique de l'alliage, présent en une quantité d'au moins 0,5% en poids de l'alliage

2. Dispositif de libération selon la revendication 1, où l'aluminium est présent dans l'alliage en une quantité dans la plage de 1 à 7,5% en poids de l'alliage.

3. Dispositif de libération selon la revendication 1 ou la revendication 2, où le cuivre est présent en une quantité jusqu'à 5% en poids de l'alliage.

4. Dispositif de libération selon l'une des revendications précédentes, où l'alliage métallique est constitué, en poids d'alliage, de
(a) une proportion majeure de magnésium;
(b) d'aluminium en une quantité inférieure à 8% en poids;
(c) de cuivre en une quantité d'au moins 0,5% en poids;
(d) de sélénium en une quantité de 0 à 0,5% en poids; et
(e) de zinc en une quantité de 0 à 5% en poids.

5. Dispositif de libération selon la revendication 4, où l'alliage métallique est constitué, en poids d'alliage, de
(a) une proportion majeure de magnésium;
(b) d'aluminium en une quantité de 1 à 7,5% en poids et
(c) de cuivre en une quantité de 0,5 à 5% en poids.

6. Dispositif de libération selon la revendication 4 ou la revendication 5, où le cuivre est présent en une quantité de 0,5 à 4% en poids de l'alliage.

7. Dispositif de libération selon l'une des revendications 4 à 6, où la quantité d'aluminium est de 2% en poids, et la quantité de cuivre est de 2-3% en poids de l'alliage.

8. Dispositif de libération selon l'une des revendications précédentes, comprenant en outre une quantité d'un matériau de poids spécifique élevé de telle sorte que le bol obtenu présente une densité d'ensemble d'au moins 2g/cm³.

9. Dispositif de libération selon la revendication 8, où le matériau de poids spécifique élevé dans le bol est un métal sous forme de poudre, de granules ou de grenailles et est sélectionné parmi le fer, le cuivre, le nickel, le zinc, le tungstène ou des alliages comprenant l'un des métaux précités.

10. Dispositif de libération selon la revendication 8 ou la revendication 9, sous la forme d'un bol comprenant un corps moulé ou extrudé oblong d'une composition comprenant ledit alliage métallique et dans lequel est dispersé le matériau de poids spécifique élevé de telle sorte que la composition d'ensemble du bol présente une densité d'au moins 2,5 g/cm².

11. Dispositif de libération selon la revendication 8, où le bol comprend un composant de gaine tubulaire en alliage de magnésium, ce composant de gaine tubulaire entoure un composant de noyau dégradable, le composant de noyau dégradable incluant un matériau électriquement conducteur de telle sorte que le composant de noyau dégradable dans son ensemble est électriquement conducteur et est couplé galvaniquement au composant de gaine tubulaire.

12. Dispositif de libération selon la revendication 11, où le composant de noyau dégradable se présente sous la forme d'un corps oblong d'une section transversale circulaire.

13. Dispositif de libération selon l'une des revendications 8 à 12, où les teneurs en aluminium et en cuivre combinées dans ledit alliage sont telles que le bol obtenu fournit un taux de libération de magnésium, comme mesuré par la procédure de test in vitro décrite dans le présent document, d'au moins 2 grammes/jour.

14. Dispositif de libération selon la revendication 11 ou la revendication 12, où le matériau électriquement conducteur dans le composant de noyau dégradable est sélectionné parmi le carbone, le graphite, la mine de plomb ou des mélanges de ceux-ci.

15. Dispositif de libération selon la revendication 11 ou la revendication 12, où le matériau de composant de noyau dégradable comporte en outre une quantité d'un matériau de poids spécifique élevé de telle sorte que le bol obtenu a une densité d'ensemble d'au moins 2 g/cm.

16. Dispositif de libération selon l'une des revendications 11, 12, 14 et 15, où le matériau de composant de noyau dégradable comporte en outre un ou plusieurs matériaux de ballast additionnels pour augmenter la solubilité du composant de noyau dans le rumen de l'animal et/ou pour contribuer à lier les composants de la composition de noyau ensemble, un ou plusieurs matériaux de ballast précités étant sélectionnés parmi le saccharose en poudre, des stéréates, de l'urée, de l'amidon, du lactose et de polymères.

17. Dispositif de libération selon l'une des revendications 8 à 16, où le bol comprend en outre un ou plusieurs agents thérapeutiques ou pharmaceutiques additionnels sélectionnés parmi l'un des suivants: des éléments de trace; des vitamines; des antibiotiques; des promoteurs de croissance; et des agents biologiquement actifs comme des vermifuges, des antiparasites, et des endectocides.

18. Utilisation, lors de la fabrication d'un dispositif de libération sous la forme d'un bol pour l'administration à un animal ruminant par le dépôt dans son sac ruméno-réticulaire, d'un alliage comprenant un alliage de magnésium/aluminium contenant une proportion majeure de magnésium, ou
(i) la quantité d'aluminium dans l'alliage est inférieure à 8% en poids; et
(ii) la quantité de cuivre dans l'alliage est au moins de 0,5% en poids, pour l'augmentation du taux de libération du magnésium.
